(19) Europäisches Patentamt
European Patent Office
Office européen des brevets

(11) **EP 4 668 288 A1**

(12) **DEMANDE DE BREVET EUROPEEN**

(43) Date de publication:
**24.12.2025 Bulletin 2025/52**

(21) Numéro de dépôt: **25175716.7**

(22) Date de dépôt: **12.05.2025**

(51) Classification Internationale des Brevets (IPC):
**G16H 50/30** (2018.01)   **A61B 5/08** (2006.01)

(52) Classification Coopérative des Brevets (CPC):
**G16H 50/30; A61B 5/08; A61B 5/0816;
A61B 5/087; A61B 5/746**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH LA MA MD TN**

(30) Priorité: **20.06.2024 FR 2406652**

(71) Demandeur: **L'AIR LIQUIDE, SOCIETE ANONYME
POUR L'ETUDE ET
L'EXPLOITATION DES PROCEDES GEORGES
CLAUDE
75007 Paris (FR)**

(72) Inventeurs:
• **RAHIM, Mehdi**
  **78354 Les Loges en Josas (FR)**
• **GUILLEMOT, Mathilde**
  **78354 Les Loges en Josas (FR)**
• **AMADOU-BOUBACAR, Habiboulaye**
  **78354 Les Loges en Josas (FR)**

(74) Mandataire: **Air Liquide**
  **L'Air Liquide S.A.
  Direction de la Propriété Intellectuelle
  75, Quai d'Orsay
  75321 Paris Cedex 07 (FR)**

(54) **SYSTÈME DE SUIVI D'UNE PERSONNE VENTILÉE DE MANIÈRE NON-INVASIVE**

(57) L'invention concerne un système de suivi d'une personne ventilée artificiellement de manière non-invasive (NIV) au moyen d'un ventilateur médical alimentant un masque respiratoire fournissant un gaz respiratoire. Le ventilateur médical fournit des variables de ventilation. Un serveur informatique reçoit les variables de ventilation et les traite pour déterminer, pour chaque variable, plusieurs indicateurs statistiques et en déduire un score de qualité de ventilation en utilisant un modèle mathématique comparant ces indicateurs statistiques et des seuils mémorisés obtenus par apprentissage-machine. On détermine enfin parmi ces variables, au moins une variable de ventilation significative impactant le score de qualité de ventilation de manière supérieure au reste des variables de ventilation.

Fig.1

EP 4 668 288 A1

**Description**

La présente invention concerne un système de suivi d'une personne ventilée artificiellement de manière non-invasive au moyen d'un ventilateur médical alimentant un masque respiratoire fournissant un gaz respiratoire, tel de l'air ou un mélange air/$O_2$ sous pression, à ladite personne en ayant besoin.

La ventilation non-invasive (VNI) est une aide mécanique à la respiration grâce à un appareil respiratoire, i.e. un ventilateur médical, qui délivre de l'air pressurisé par l'intermédiaire d'un masque appliqué sur le visage d'une personne à ventiler artificiellement, typiquement un patient.

La VNI est un traitement reconnu des insuffisances respiratoires hypercapniques chroniques. Ce traitement permet d'améliorer les symptômes en diminuant le travail des muscles respiratoires et en améliorant les échanges gazeux.

De plus, il peut être mis en œuvre facilement au domicile du patient, c'est-à-dire hors hôpital. Dès lors, dans le cadre d'un traitement long, la VNI peut permettre une diminution du nombre d'hospitalisation des patients insuffisants respiratoires.

Dans tous les cas, le succès de la VNI au long cours est conditionné par l'acceptation et la participation du patient et de son entourage au traitement. Il est donc nécessaire d'opérer un suivi du patient à distance, en particulier lorsqu'il est traité à son domicile, c'est-à-dire hors hôpital.

Le monitoring ou suivi du traitement du patient peut être opéré par analyse de données de télémétrie transmises par le ventilateur médical lorsqu'il est connecté, ainsi que par le recueil du ressenti du patient qui est évalué avec un (ou des) questionnaire, par exemple celui nommé « S3 -NIV » qui comprend 11 questions portant sur les symptômes respiratoires, la qualité du sommeil et les effets secondaires du traitement par VNI, de sorte de de déterminer un score de traitement pour chaque patient.

Si un score est un outil très utile dans le suivi d'un patient ventilé, il nécessite toutefois une intervention humaine (i.e. intervention d'un personnel soignant au domicile du patient) afin de pouvoir être récolté. Ce qui est contraignant au plan opérationnel et dès lors difficile à déployer à large échelle.

Au vu de cela, un problème est de pouvoir réaliser un suivi ou monitoring efficace à distance d'un patient traité par VNI à son domicile, lequel ne requiert pas d'intervention humaine et permette de préférence de prédire le score de qualité de ventilation du patient à partir des données de télémétries fournies par le ventilateur délivrant le traitement de VNI au patient ventilé considéré.

Une solution de l'invention concerne alors un système de suivi d'une personne ventilée artificiellement, i. un patient, de manière non-invasive (NIV) au moyen d'un ventilateur médical alimentant un masque respiratoire ou analogue fournissant un gaz respiratoire, tel de l'air ou un mélange air/$O_2$, à ladite personne, comprenant :

- le ventilateur médical configuré pour fournir des variables de ventilation choisies parmi une observance, des fuites au masque, un indice d'apnée/hypopnée (i.e. IAH), une fréquence respiratoire, un taux de respiration spontanée (%) et un volume courant (i.e. *tidal volume*), et
- au moins un serveur informatique configuré pour recevoir les variables de ventilation fournies par le ventilateur médical et pour les traiter.

De, plus, ledit au moins un serveur informatique du système de suivi de l'invention est configuré pour :

a) traiter les variables de ventilation en :

i) déterminant pour chaque variable de ventilation, plusieurs indicateurs statistiques choisis parmi une moyenne, un écart-type, une excentricité (i.e. skewness), un aplatissement (i.e. kurtosis), une tendance, une variance, une médiane et une amplitude (i.e. max-min),
ii) calculant un score de qualité de ventilation à partir desdits indicateurs statistiques en utilisant un modèle mathématique permettant d'opérer une comparaison desdits indicateurs statistiques à des seuils mémorisés obtenus par apprentissage-machine (i.e. Machine Learning), et
iii) déterminant parmi lesdites variables de ventilation, au moins une variable de ventilation significative impactant le score de qualité de ventilation de manière supérieure au reste des variables de ventilation,

b) commander un affichage sur des moyens d'affichage, du score de qualité de ventilation déterminé en a) ii) et de ladite au moins une variable de ventilation significative, et
c) générer une alerte de qualité de ventilation lorsque le score de qualité de ventilation est inférieur à une valeur de seuil prédéfini mémorisée au sein du serveur informatique.

Selon le mode de réalisation considéré, l'invention peut comprendre l'une ou plusieurs des caractéristiques suivantes :

- le ventilateur médical comprend une soufflante (i.e. turbine, compresseur ou analogue) délivrant le gaz respiratoire.
- le gaz respiratoire est de l'air, typiquement de l'air sous pression (>1 atm).
- les variables de ventilation sont des données « machine » provenant du ventilateur médical.
- les indicateurs statistiques comprennent préférentiellement une moyenne, un écart-type, un excentricité (i.e. skewness), aplatissement (i.e. kurtosis) et une tendance.
- les variables de ventilation comprennent l'observance et au moins une autre variable choisie(s) parmi les fuites au masque, l'indice d'apnée/hypopnée (i.e. IAH) et la fréquence respiratoire.
- il comprend en outre des moyens d'alerte configurés pour déclencher une alerte lorsque le score de qualité de ventilation est supérieur à une valeur de seuil prédéterminée, et ledit au moins un serveur informatique coopère avec le dispositif d'affichage pour afficher l'alerte ayant été déclenchée par les moyens d'alerte.
- le modèle mathématique comprend un algorithme d'apprentissage.
- le dispositif d'affichage est configuré pour afficher simultanément le score de qualité de ventilation et au moins une variable de ventilation significative impactant ledit score de qualité de ventilation de manière supérieure aux autres (i.e. au reste des) variables de ventilation.
- le dispositif d'affichage est configuré pour afficher une liste de plusieurs patients classés du score de ventilation le plus faible au plus élevé.
- le dispositif d'affichage est configuré pour afficher les patients regroupés en fonction de leurs scores de qualité de ventilation.
- les groupes de patients sont affichés dans des couleurs différentes.
- le dispositif d'affichage est configuré pour afficher une représentation graphique d'au moins une variable de ventilation d'un patient considéré sous forme d'une courbe ou d'un barographe sur plusieurs jours.
- les moyens informatiques de traitement de données sont configurés pour traiter les mesures de variables de ventilation obtenues sur une période temporelle de 4 à 30 jours, de préférence de 4 à 15 jours.
- il comprend des moyens d'alerte configurés pour générer l'alerte de qualité de ventilation.
- les moyens d'alerte sont configurés pour déclencher une alerte sonore ou visuelle chez un professionnel de santé opérant le suivi du patient à distance (PSAD).
- il comprend un dispositif de mesure configuré pour opérer des mesures relatives au flux gazeux, en particulier des mesures de pression et de débit.
- le dispositif de mesure est configuré pour déterminer les variables de ventilation à partir des mesures opérées, en particulier des mesures de pression et de débit.
- le dispositif de mesure comprend des moyens traitement de données, en particulier un (ou des) microprocesseur, de préférence agencé sur une (des) carte électronique.
- le dispositif de mesure est agencé sur le trajet du gaz, c'est-à-dire typiquement entre la soufflante et le masque respiratoire.
- le dispositif de mesure est préférentiellement intégré au ventilateur.
- alternativement, le dispositif de mesure est agencé sur un conduit reliant fluidiquement le ventilateur au masque respiratoire, tel un tuyau flexible.
- le dispositif de mesure peut comprend aussi des moyens de télétransmission de données configurés pour transmettre à distance au moins les variables de ventilation calculées par le dispositif de mesure.
- alternativement, les moyens de télétransmission sont intégrés au ventilateur et coopèrent avec le dispositif de mesure, en particulier avec les moyens traitement de données dudit dispositif de mesure.

L'invention va maintenant être mieux comprise grâce à la description détaillée suivante, faite à titre illustratif mais non limitatif, en référence aux figures annexées parmi lesquelles :

- Fig. 1 schématise un mode de réalisation d'un système selon l'invention.
- Fig. 2 schématise un mode de réalisation d'une chaîne de décision permettant de prédire le score de qualité de ventilation et d'en déterminer les causes, mis en œuvre par un système selon l'invention, et
- Fig. 3 schématise un mode de réalisation des moyens d'affichage et de visualisation des scores de qualité de ventilation, variables de ventilation et causes de qualité de ventilation, mis en œuvre par un système selon l'invention.

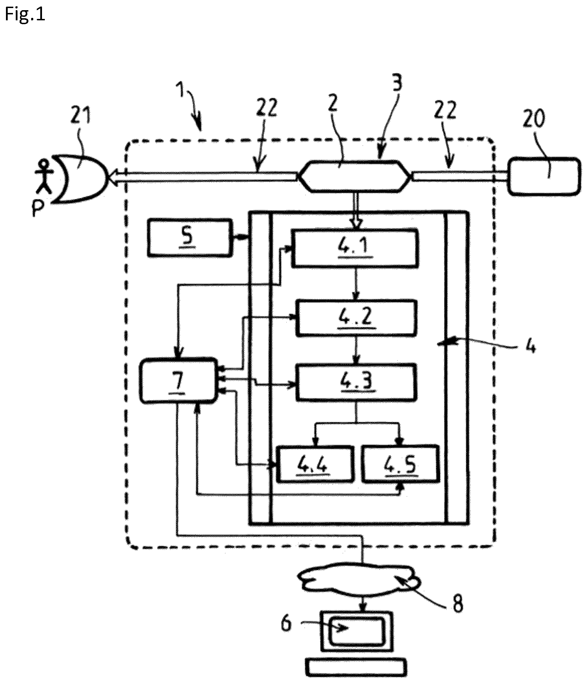

Sur Fig. 1, on a schématisé un mode de réalisation d'un système de suivi 1 ou monitoring selon l'invention permettant de soigner un patient P souffrant d'insuffisances respiratoires hypercapniques chroniques, par insufflation de gaz sous pression, tel de l'air ou un mélange air/$O_2$, délivré par un ventilateur médical 20, i.e. un appareil d'assistance respiratoire. Le gaz sous pression est acheminé par un conduit flexible 22 depuis le ventilateur médical 20 jusqu'à un masque respiratoire 21 délivrant le gaz aux voies aériennes du patient P, tel un masque nasal ou analogue.

Le système 1 de l'invention permet de calculer un score de qualité de ventilation et d'alerter sur un risque d'un mauvais ressenti de ventilation du patient P à son traitement. Ce système 1 comprend un dispositif de mesure 2 est agencé sur le trajet du gaz, par exemple comme montré ici sur le conduit flexible 22 reliant le ventilateur 20 au masque 21, afin de permettre d'y opérer des mesures en lien avec le flux gazeux, en particulier de pression et/ou de débit.

Selon un autre mode de réalisation (non montré), le dispositif de mesure 2 peut être agencé directement dans le ventilateur médical 20, c'est-à-dire en sortie de soufflante.

Le dispositif de mesure 2 comprend aussi des moyens de traitement de données, typiquement un (des) microprocesseur porté par une carte électronique, configurés pour déterminer, à partir des mesures opérées (i.e. pression et débit du gaz), des valeurs de plusieurs variables de ventilation ($p_1$, $p_2$..., $p_i$) relatifs au patient P, à savoir l'observance, les fuites au masque, l'indice d'apnée/hypopnée (IAH), la fréquence respiratoire, un taux de respiration spontanée, le volume courant, et éventuellement d'autres variables ou paramètres.

Calculer de telles variables de ventilation est connu en soi, notamment de Manel Luján, Cristina Lalmolda, and Begüm Ergan, 'Basic Concepts for Tidal Volume and Leakage Estimation in Non-Invasive Ventilation', Turkish Thoracic Journal 20, no. 2 (April 2019): 140-46, https://doi.org/10.5152/TurkThoracJ.2018.177, ou Jean-Michel Arnal, Mathilde Oranger, and Jésus Gonzalez-Bermejo, 'Monitoring Systems in Home Ventilation', Journal of Clinical Medicine 12, no. 6 (10 March 2023): 2163, https://doi.org/10.3390/jcm12062163.

Plus précisément, dans le mode de réalisation représenté, le dispositif de mesure 2 comprend un boîtier 3 incluant des moyens de mesure adaptés, tels un ou des capteurs de débit, de pression ou autre, permettant d'opérer les différentes mesures désirées, les moyens de traitement de données permettant de traiter ces mesures pour en déduire les variables de ventilation, ainsi qu'une source de courant interne, telle qu'une pile ou batterie, alimentant notamment les moyens de mesure ; alternativement, l'alimentation électrique est fournie par le ventilateur 20.

On prévoit par ailleurs des moyens de télétransmission de données configurés pour transmettre à distance au moins les variables de ventilation fournies par le dispositif de mesure 2. Par exemple, ces moyens de télétransmission comprennent un modem de type GSM ou autre et une antenne émettrice. Les variables transmises transitent préférentiellement via le réseau internet ou un autre réseau, tels que les réseaux GSM, Lora ou Sigfox, et/ou peuvent être traités dans l'espace informatique virtuel ou "cloud".

On prévoit en outre des moyens informatiques de traitement de données, à savoir (au moins) un serveur informatique 4, comprenant des moyens de réception de données configurés pour recevoir des variables de ventilation télétransmises par les moyens de transmission de données, ainsi qu'un ou plusieurs processeurs permettant de traiter les valeurs des variables de ventilation mesurées par le dispositif de mesure 2 et en déduire des caractéristiques représentatives ($c_1$,$c_2$...,$c_j$) des variables de ventilation du patient P, lesquelles sont ensuite comparées à des valeurs-seuils ($S_1$, $S_2$,...$S_k$) mémorisées au sein de moyens de mémorisation 7, comme une mémoire (e.g. disque dur, mémoire flash, stockage cloud ... ) ou autre, afin d'en déduire un score de qualité de ventilation pour le patient P compris entre 0 et 100%.

Il est à noter que, selon un autre mode de réalisation (non montré), les variables de ventilation déterminées par le dispositif de mesure 2 peuvent être aussi traitées par les moyens de traitement de données dudit dispositif de mesure 2 afin d'en déduire les caractéristiques représentatives ($c_1$,$c_2$...,$c_j$) des variables de ventilation du patient P, avant que celles-ci soient transmises au serveur informatique 4.

Les moyens informatiques de traitement de données ou serveur 4 sont alimentés électriquement par des moyens de fourniture de courant électrique 5, tel le réseau électrique (110/220V) ou une (ou des) pile(s) ou batterie(s) de stockage.

Par ailleurs, le système 1 de l'invention comprend aussi des moyens d'affichage, aussi appelés dispositif d'affichage 6, permettant d'afficher des informations, des données... par exemple un écran d'affichage d'un ordinateur fixe ou portable, d'un téléphone multifonction (smartphone), d'une tablette numérique ou autre.

Le dispositif d'affichage 6 reçoit les données à afficher via un système ou des moyens de communication 8 adaptés, par exemple des moyens de transmission de données configurés pour transmettre les données ou autres informations, via un protocole de communication, tel que le wifi, le GSM (3G/4G/5G), le Bluetooth®, l'internet, un intranet ou autre.

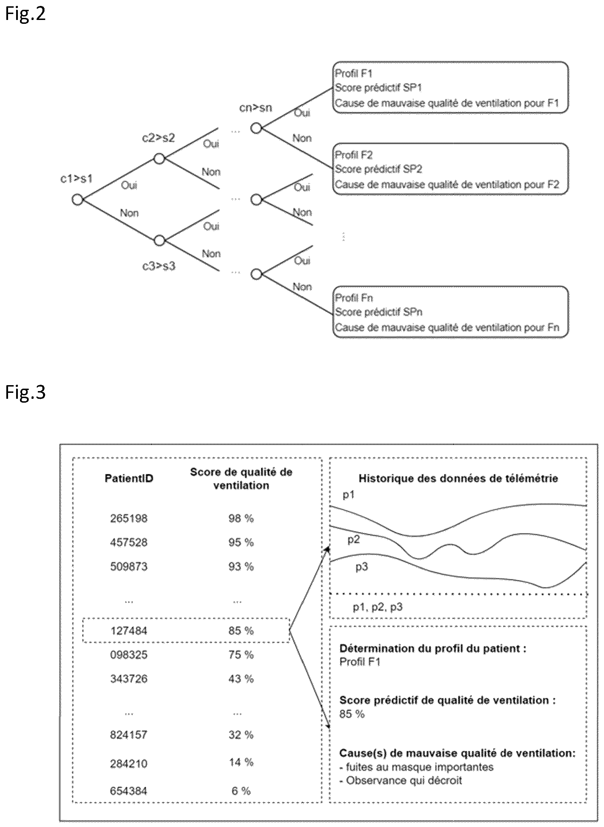

Fig. 2 schématise un mode de réalisation de la chaîne décisionnelle mise e œuvre au sein du serveur 4 permettant de prédire le score de qualité de ventilation du patient P et d'en identifier les causes, c'est-à-dire la chaîne décisionnelle mise en œuvre par le système selon l'invention, tel celui de Fig. 1.

Une fois que le dispositif de mesure 2 du système de l'invention 1 a opéré des mesures de débit et/ou de pression du gaz respiratoire fourni au patient, tel de l'air, pendant une durée de plusieurs, à savoir avantageusement d'au moins 4 jours, préférentiellement entre 4 et 15 jours, les moyens informatiques de traitement de données 4, tel un serveur distant, traitent ces mesures de débit et/ou de pression obtenues sur au moins 4 jours pour déterminer ou estimer des grandeurs ou valeurs de plusieurs variables de ventilation $p_1$, $p_2$..., $p_i$ du patient P.

Les variables de ventilation $p_1$, $p_2$..., $p_i$ ainsi déterminées sont (au moins) l'observance, les fuites au masque, l'indice d'apnée/hypopnée, la fréquence respiratoire, un taux de respiration spontanée et le volume courant.

Ces grandeurs ou valeurs de plusieurs variables de ventilation $p_1$, $p_2$..., $p_i$ sont utilisées ensuite par le serveur 4, pour en déduire des indicateurs $c_1$, $c_2$...,$c_n$ liés à un score de qualité de ventilation et les comparer à des valeurs-seuils $s_1$, $s_2$,...,$s_n$ mémorisées de manière à évaluer un profil spécifique pour le patient P.

Ce profil spécifique correspond en fait à l'un de plusieurs profils F1, F2, ..., Fn mémorisés, qui sont liés chacun à un score prédictif SP de qualité de ventilation ressentie donné, i.e. SP1, SP2...SPn.

Ces différents profils F1, F2, ..., Fn prédéterminés, scores prédictifs SP1, SP2...SPn liés et les valeurs-seuils $s_1$, $s_2$,..., $s_k$ sont mémorisés par des moyens de mémorisation, de préférence par le serveur lui-même. Ils ont été obtenus à partir d'un historique de plusieurs patients dont l'adhérence au traitement est connue.

Chaque profil mémorisé F1, F2, ..., Fn est caractérisé par (au moins) un score prédictif SP de qualité de ventilation.

Autrement dit, en procédant ainsi, grâce aux différents profils F1, F2, ..., Fn prédéterminés, c'est-à-dire connus et enregistrés, il est possible de déterminer aisément à quel profil exact correspond le profil spécifique du patient P considéré et donc quel est le score prédictif SP de qualité de ventilation ressentie de ce patient qui est typiquement compris entre 0 et 100%.

Ensuite, comme illustré en Fig. 3, le dispositif d'affichage 6 du système 1 de l'invention est configuré pour afficher le score de qualité de ventilation (en %) pour le ou les patients considérés, par exemple ici pour au moins 9 patients identifiés chacun par une identification propre (PatientID).

De plus, le dispositif d'affichage 6 du système 1 de l'invention est aussi configuré pour afficher une représentation graphique, telle une ou des courbes en fonction du temps, d'une (ou plusieurs) variable de ventilation ($p_1$, $p_2$..., $p_i$) d'un patient sélectionné parmi l'ensemble des patients affichés.

Pour ce faire, un utilisateur, typiquement un personnel soignant, sélectionne le patient particulier pour lequel il souhaite avoir plus d'informations, en particulier pouvoir visualiser l'évolution de ce (ou ces) variables de ventilation ($p_1$, $p_2$..., pi). Lorsque cette sélection est opérée, le dispositif d'affichage 6 opère l'affichage désiré de la représentation graphique, par exemple ici des courbes de plusieurs variables de ventilation du patient faisant office d'historique de mesures sur plusieurs jours, typiquement entre 4 et 30 jours.

La représentation graphique est préférentiellement associée à d'autres informations du patient, notamment son identification (PatientID), son score de qualité de ventilation (%), et avantageusement une ou des causes de mauvaise qualité de ventilation déterminées pour le patient considéré.

Les causes de mauvaise qualité de ventilation sont préférentiellement des fuites au masque ou un réglage de pression inadéquat, mais aussi d'autres variables de ventilation. Les causes de mauvaise qualité de ventilation sont déterminées par les moyens informatiques de traitement de données 4 à partir du score de ventilation obtenu pour le patient considéré.

En fait, chaque profil mémorisé est caractérisé par (au moins) un score prédictif SP de qualité de ventilation ressentie et une cause de mauvaise qualité de ventilation.

Avantageusement, pour faciliter la lecture et permettre à l'utilisateur, c'est-à-dire le personnel soignant, de distinguer rapidement les patients avec les moins bonnes qualités de ventilation, le dispositif d'affichage 6 du système 1 de l'invention est aussi configuré pour afficher les patients en une liste de plusieurs patients classés or organisés en fonction du score de qualité de ventilation, c'est-à-dire par priorité, i.e. du plus élevé (en tête de liste) au moins élevé (en bas de liste), comme illustré en Fig. 3.

La description suivante permet de mieux comprendre l'invention.

Comme illustré en Fig. 1, le serveur 4 récupère et prétraite (en 4.1) d'abord les variables de ventilation ($p_1$, $p_2$..., $p_i$) relatifs au patient P, telle que l'observance, les fuites au masque, l'indice d'apnée/hypopnée, la fréquence respiratoire, un taux de respiration spontanée et le volume courant,

Par exemple, le prétraitement (4.1) peut comprendre des étapes de :

a) Mise à l'échelle des valeurs des variables de ventilation ($p_1$, $p_2$..., $p_i$) en fonction du ventilateur utilisé et de ses caractéristiques intrinsèques, par exemple une fuite au masque médiane ou moyenne, grandeurs exprimées en % ou en valeur absolue.... Ainsi, pour une variable de ventilation p_i et d'une machine donnée m, on obtient : $q\_i(m) = p\_i(m)$ - *moyenne* ($p\_i,m$) / $std(p\_i,m)$ où :

- moyenne(p_i, m) est la moyenne empirique de la variable de ventilation pi pour la machine m considérée (i.e. selon le constructeur).
- std(p_i, m) est l'écart-type de la variable de ventilation p_i pour la machine m considérée.
- q_i est la valeur de la variable de ventilation mise à l'échelle.

b) Remplacement des valeurs manquantes dans les variables de ventilation ($p_1$, $p_2$..., $p_i$). Pour une variable de ventilation p_i dont la valeur est manquante à un point t, plusieurs méthodes peuvent être appliquées:

- Imputation à valeur fixe: p_i(t) = 0
- Imputation par la moyenne: p_i(t) = moyenne(pi)
- Imputation par la valeur précédente: p_i(t) = p_i(t-1).

Ensuite, ces variables de ventilation ($p_1$, $p_2$..., $p_i$) sont traités et on en déduit les caractéristiques représentatives ($c_1$, $c_2$...,$c_j$), aussi appelées indicateurs statistiques, qui sont enfin comparées (en 4.2) aux valeurs-seuils ($s_1$, $s_2$...$s_k$) mémorisées (en 7) pour déterminer (en 4.3) le score de qualité de ventilation.

Par exemple, le traitement (4.2) comprend des étapes de calcul d'indicateurs statistiques sur les variables de ventilation $p_i$ (observance, les fuites au masque, l'indice d'apnée/hypopnée (IAH), la fréquence respiratoire, un taux de respiration spontanée (%) et le volume courant (tidal volume), par exemple détermination de moyenne, tendance ... comme donné dans le Tableau suivant.

<div align="center">Tableau</div>

| | Caractéristiques représentatives (C1, C2... ,$c_j$) | Description |
|---|---|---|
| Informations patient | Genre | Homme, Femme |
| | Âge | Âge du patient |
| | Durée | Durée du traitement |
| Variables de ventilation pi | Fuites | Moyenne |
| | | Ecart type |
| | | Excentricité (asymétrie) |
| | | Aplatissement |
| | | Tendance |
| | | Excentricité (asymétrie) |
| | Observance | Moyenne |
| | | Ecart type |
| | | Excentricité (asymétrie) |
| | | Aplatissement |
| | | Tendance |
| | IAH | Moyenne |
| | | Ecart type |
| | | Excentricité (asymétrie) |
| | | Aplatissement |
| | | Tendance |
| | Fréquence respiratoire | Moyenne |
| | | Ecart type |
| | | Excentricité (asymétrie) |
| | | Aplatissement |
| | | Tendance |
| | Taux de respiration spontanée | Moyenne |
| | | Ecart type |
| | | Excentricité (asymétrie) |
| | | Aplatissement |
| | | Tendance |

(suite)

|  | Caractéristiques représentatives (C1, C2... ,c_j) | Description |
|---|---|---|
|  | Volume courant | Moyenne |
|  |  | Ecart type |
|  |  | Excentricité (asymétrie) |
|  |  | Aplatissement |
|  |  | Tendance |

Ces indicateurs statistiques sont calculés par exemple via les formules suivantes où D est le nombre total de jours t, et X(t) est la variable de ventilation relevée à un jour t donné. Ainsi :

La moyenne $\mu$ est calculée à partir de l'équation : $\mu = (1/D) . \Sigma_t X(t)$
L'écart-type $\sigma$ est calculé à partir de l'équation : $\sigma = (1/D) . \Sigma_t (X - \mu)^2$
La variance V correspond à : $V = \sigma^2$
L'excentricité S (l'asymétrie) correspond à : $S = \mu^3 / \sigma^3$
L'aplatissement correspond à : $S = \mu^4 / \sigma^4$
La tendance â correspond à : â , $\hat{B} = \text{argmin} \sum_t (X(t) - (at + b))^2$

Le score de qualité de ventilation obtenu sert à déclencher une alerte de mauvaise qualité de ventilation (en 4.4) pour le patient considéré.

L'alerte de qualité de ventilation (4.4) peut être transmise au dispositif d'affichage 6, tel un écran d'ordinateur ou de téléphone multifonction (smartphone), qui permet de l'afficher. La transmission du score SP peut se faire via un protocole et/ou réseau de communication 8, tel que web, GSM ou autre.

Le déclenchement d'une alerte de qualité de ventilation (en 4.4) se fait préférentiellement via un processus de Machine Learning (apprentissage machine).

Ce processus utilise un historique de données comprenant au moins les variables de ventilation préalablement collectées sur des patients dont on connaît l'issue, c'est-à-dire pour lequel on connaît le score S3-niv, après la période cible de 4 à 30 jours, typiquement 15 jours.

Une méthode mathématique utilisable, comme illustré en Fig. 2, repose sur une priorisation des caractéristiques représentatives $(c_1, c_2...,c_i)$ extraites des variables de ventilation, de sorte que les premières soient celles ayant un pouvoir discriminant plus important. Plus précisément, une caractéristique c est choisie comme prioritaire si elle permet de séparer à l'aide d'un seuil s, le plus grand nombre de patients en deux groupes, à savoir un groupe de patients ayant majoritairement une bonne qualité de ventilation et un groupe de patients ayant majoritairement une mauvaise qualité de ventilation.

La détermination de la caractéristique prioritaire c et le choix optimal du seuil s sont effectués avec un algorithme d'optimisation, comme celui décrit par : Leo Breiman, Classification and regression trees, Monterey, CA, Wadsworth & Brooks/Cole Advanced Books & Software, 1984, 368 p. (ISBN 978-0-412-04841-8)

Cette méthode est appliquée séquentiellement à chaque groupe ainsi obtenu pour déterminer la caractéristique prioritaire et son seuil optimal de ces groupes, et obtenir alors 2 nouveaux sous-groupes. Ceci est répété plusieurs fois de suite.

Le processus s'arrête avec un critère d'arrêt basé sur le nombre d'itérations ou lorsque les sous-groupes de patients correspondent à des profils suffisamment homogènes de patients, c'est-à-dire des patients qui ont principalement une bonne qualité de ventilation ou principalement une mauvaise qualité de ventilation. Ces profils suffisamment homogènes comprennent tous les profils Fn, i.e. F1, F2, ..., Fn.

Le score prédictif SP_n associé à chaque profil Fn est calculé comme étant la proportion des patients avec une mauvaise qualité de ventilation ressentie suivant la formule:

SP_n(Profil Fn) = Nb-P mauvaise_qualité_ventilation (Profil Fn) / Nb-P (Profil Fn)

où : Nb-P est le nombre de patients

Enfin, les patients de chaque profil Fn de la base de données étant préalablement bien connus par le personnel soignant, les causes principales de leur mauvaise qualité de ventilation sont généralement connues car communes à tous les patients d'un même profil (Fn).

De plus, pour déterminer pour chaque prédiction les facteurs les plus importants, c'est-à-dire ceux ayant conduit au calcul du score de qualité de ventilation, on peut utiliser un ou des algorithmes d'apprentissage machine ou Machine Learning, par exemple ceux décrits par :

- M.T. Ribeiro, S. Singh, C. Guestrin, Anchors: high-precision model-agnostic explanations, AAAI conference on artificial intelligence (AAAI); 2018, *ou*
- M.T. Ribeiro, S. Singh, C. Guestrin, why should I trust you?" explaining the predictions of any classifier, Proceedings of the 22nd ACM SIGKDD international conference on knowledge discovery and data mining; 2016, pp. 1135-1144.

L'alerte de non-adhérence peut être visuelle et/ou sonore. A cette fin, le système de l'invention peut comprendre des moyens d'affichage visuel et/ou des moyens sonores.

D'une façon plus générale, le système de l'invention permet de calculer un score de qualité de ventilation et d'alerter d'un risque de mauvais ressenti de la ventilation d'un patient P souffrant d'insuffisance respiratoire hypercapnique chronique, ledit patient P étant traité par administration de gaz sous pression, tel de l'air ou un mélange d'air/$O_2$, délivré par un appareil d'assistance respiratoire de type ventilateur médical.

**Revendications**

1. Système de suivi (1) d'une personne (P) ventilée artificiellement de manière non-invasive (NIV) au moyen d'un ventilateur médical (20) alimentant un masque respiratoire (21) fournissant un gaz respiratoire à ladite personne (P), comprenant :

   - le ventilateur médical (20) configuré pour fournir des variables de ventilation choisies parmi observance, fuites au masque, indice d'apnée/hypopnée, fréquence respiratoire, taux de respiration spontanée et volume courant, et
   - au moins un serveur informatique (4) configuré pour recevoir les variables de ventilation fournies par le ventilateur médical et pour les traiter,

   **caractérisé en ce que** ledit au moins un serveur informatique (4) est configuré pour:

   a) traiter les variables de ventilation en :

      i) déterminant pour chaque variable de ventilation, plusieurs indicateurs statistiques choisis parmi une moyenne, un écart-type, une excentricité, un aplatissement, une tendance, une variance, une médiane et une amplitude,
      ii) calculant un score de qualité de ventilation à partir desdits indicateurs statistiques en utilisant un modèle mathématique permettant d'opérer une comparaison desdits indicateurs statistiques à des seuils mémorisés obtenus par apprentissage-machine, et
      iii) déterminant parmi lesdites variables de ventilation, au moins une variable de ventilation significative impactant le score de qualité de ventilation de manière supérieure au reste des variables de ventilation,

   b) commander un affichage sur des moyens d'affichage, du score de qualité de ventilation déterminé en a) ii) et de ladite au moins une variable de ventilation significative, et
   c) générer une alerte de qualité de ventilation lorsque le score de qualité de ventilation est inférieur à une valeur de seuil prédéfini mémorisée au sein du serveur informatique.

2. Système selon la revendication 1, **caractérisé en ce que** les indicateurs statistiques comprennent une moyenne, un écart-type, un excentricité, aplatissement et une tendance.

3. Système selon la revendication 1, **caractérisé en ce que** les variables de ventilation comprennent l'observance et au moins une autre variable choisie parmi les fuites au masque, l'indice d'apnée/hypopnée et la fréquence respiratoire.

4. Système selon la revendication 1, **caractérisé en ce qu'**il comprend en outre des moyens d'alerte configurés pour déclencher une alerte lorsque le score de qualité de ventilation est supérieur à une valeur de seuil prédéterminée, et ledit au moins un serveur informatique coopère avec le dispositif d'affichage pour afficher l'alerte ayant été déclenchée par les moyens d'alerte.

5. Système selon la revendication 1, **caractérisé en ce que** le modèle mathématique comprend un algorithme d'apprentissage.

6. Système selon la revendication 1, **caractérisé en ce que** le dispositif d'affichage est configuré pour afficher simultanément le score de qualité de ventilation et au moins une variable de ventilation significative impactant ledit score de qualité de ventilation de manière supérieure aux autres variables de ventilation.

7. Système selon la revendication 6, **caractérisé en ce que** le dispositif d'affichage est configuré pour afficher une liste de plusieurs patients classés du score de ventilation le plus faible au plus élevé.

8. Système selon la revendication 7, **caractérisé en ce que** le dispositif d'affichage est configuré pour afficher les patients regroupés en fonction de leurs scores de qualité de ventilation, de préférence les groupes de patients sont affichés dans des couleurs différentes.

9. Système selon la revendication 6, **caractérisé en ce que** le dispositif d'affichage est configuré pour afficher une représentation graphique d'au moins une variable de ventilation d'un patient considéré sous forme d'une courbe ou d'un barographe sur plusieurs jours.

10. Système selon la revendication 1, **caractérisé en ce que** les moyens informatiques de traitement de données sont configurés pour traiter les mesures de variables de ventilation obtenues sur une période temporelle de 4 à 30 jours.

11. Système selon la revendication 1, **caractérisé en ce qu'**il comprend des moyens d'alerte configurés pour déclencher une alerte sonore ou visuelle chez un professionnel de santé opérant le suivi du patient à distance (PSAD)

12. Système selon la revendication 10, **caractérisé en ce que** la période temporelle est de 4 à 15 jours.

13. Système selon la revendication 1, **caractérisé en ce qu'**un dispositif de mesure (2) est configuré pour opérer des mesures du gaz, en particulier des mesures de pression et/ou de débit.

14. Système selon la revendication 13, **caractérisé en ce que** le dispositif de mesure (2) est configuré pour déterminer les variables de ventilation à partir des mesures opérées.

15. Système selon la revendication 1, **caractérisé en ce que** le ventilateur médical (20) comprend une soufflante délivrant le gaz respiratoire, de préférence le gaz respiratoire est de l'air.

Fig.1

Fig.2

Fig.3

Europäisches
Patentamt

European
Patent Office

Office européen
des brevets

# RAPPORT DE RECHERCHE EUROPEENNE

**Numéro de la demande**

EP 25 17 5716

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 2022/020488 A1 (KENNEDY COLIN BRADLEY [US] ET AL) 20 janvier 2022 (2022-01-20) * alinéas [0012] - [0025] * ----- | 1-15 | INV. G16H50/30 A61B5/08 |
| A | EP 3 753 477 A1 (PHARMA DOM [FR]; ALEHOS DEV [FR]; VITALAIRE [FR]; AIR LIQUIDE [FR]) 23 décembre 2020 (2020-12-23) * alinéas [0021], [0219] - [0233] * ----- | 1-15 | |
| A | US 2021/251512 A1 (VERHOEVEN JAN [DE] ET AL) 19 août 2021 (2021-08-19) * alinéas [0022] - [0083] * ----- | 1-15 | |

**DOMAINES TECHNIQUES RECHERCHES (IPC)**

G16H
A61B

Le présent rapport a été établi pour toutes les revendications

| Lieu de la recherche | Date d'achèvement de la recherche | Examinateur |
|---|---|---|
| Munich | 16 octobre 2025 | Rivera Pons, Carlos |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
........................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 25 17 5716

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-10-2025

| Document brevet cité au rapport de recherche | Date de publication | Membre(s) de la famille de brevet(s) | Date de publication |
|---|---|---|---|
| US 2022020488 A1 | 20-01-2022 | CN 113382676 A | 10-09-2021 |
| | | EP 3873329 A2 | 08-09-2021 |
| | | EP 4570290 A2 | 18-06-2025 |
| | | JP 7500559 B2 | 17-06-2024 |
| | | JP 2022506805 A | 17-01-2022 |
| | | US 2022020488 A1 | 20-01-2022 |
| | | WO 2020092701 A2 | 07-05-2020 |
| EP 3753477 A1 | 23-12-2020 | EP 3753477 A1 | 23-12-2020 |
| | | FR 3097424 A1 | 25-12-2020 |
| US 2021251512 A1 | 19-08-2021 | DE 102021000315 A1 | 19-08-2021 |
| | | EP 3865163 A1 | 18-08-2021 |
| | | US 2021251512 A1 | 19-08-2021 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MANEL LUJÁN** ; **CRISTINA LALMOLDA** ; **BEGÜM ERGAN**. Basic Concepts for Tidal Volume and Leakage Estimation in Non-Invasive Ventilation. *Turkish Thoracic Journal*, April 2019, vol. 20 (2), 140-46, https://doi.org/10.5152/TurkThoracJ.2018.177

- **JEAN-MICHEL ARNAL** ; **MATHILDE ORANGER** ; **JÉSUS GONZALEZ-BERMEJO**. Monitoring Systems in Home Ventilation. *Journal of Clinical Medicine*, 10 March 2023, vol. 12 (6), 2163, https://doi.org/10.3390/jcm12062163

- Classification and regression trees, Monterey, CA. **LEO BREIMAN**. Cole Advanced Books & Software. Wadsworth & Brooks, 1984, 368

- **M.T. RIBEIRO** ; **S. SINGH** ; **C. GUESTRIN**. Anchors: high-precision model-agnostic explanations. *AAAI conference on artificial intelligence (AAAI);*, 2018

- **M.T. RIBEIRO** ; **S. SINGH** ; **C. GUESTRIN**. why should I trust you?'' explaining the predictions of any classifier. *Proceedings of the 22nd ACM SIGKDD international conference on knowledge discovery and data mining*, 2016, 1135-1144